# EUROPEAN PATENT APPLICATION

(11) **EP 4 527 396 A1**
(43) Date of publication of application: **26.03.2025**
(21) Application number: 23382960.5
(22) Date of filing: 22.09.2023
(51) Int. Cl.: A61K 35/66, A61K 35/742, A61K 35/744, A61K 35/745, A61K 35/747, A61K 36/064, A61P 25/00, A61P 25/18, A61P 25/20, A61P 25/22, A61P 25/24

(54) **THERAPEUTIC USE OF A POSTBIOTIC COMPOSITION FOR PSYCHIATRIC DISORDERS**

(71) Applicant: Igen Biolab Group AG, 6300 Zug (CH)
(72) Inventor: MOSCOSO DEL PRADO UCELAY, Juan, 28760 Tres Cantos (ES); GUARDIA ALBA, María Jesús, 18198 HUETOR VEGA (ES)
(74) Representative: Padial Martinez, Ana Belen

(57) **Abstract**

Postbiotic composition for use in the treatment of psychiatric disorders in a subject, comprising microorganisms of the following genera: *Bacillus, Bifidobacterium, Lactobacillus, Saccharomyces* and *Streptococcus,* for topical or oral administration, including a pharmaceutical formulation.

## Description

### TECHNICAL FIELD OF THE INVENTION

The present invention describes a postbiotic composition that comprises lysates of probiotic microorganisms for use as a treatment and/or prevention for psychiatric disorders

### BACKGROUND

The discovery of the size and complexity of the human microbiome has resulted in an on-going evaluation of many concepts of health and disease. Certainly, dramatic changes in microbiota composition have been documented in gastrointestinal disorders such as inflammatory bowel disease (IBD). More recently, there is increased interest in the art regarding alternations in the gut microbiome that may play a pathophysiological role in human brain diseases. Preclinical and clinical evidence are strongly suggesting a link between brain development and microbiota.

A growing body of preclinical literature has demonstrated bidirectional signalling between the brain and the gut microbiome, involving multiple neurocrine and endocrine signalling systems. Indeed, increased levels of *Clostridium* species in the microbiome have been linked to brain disorders, and an imbalance of the *Bacteroidetes* and *Firmicutes* phyla has also been implicated in brain development disorders. This indicates that, at present, the practical effect of the link between the microbiome and human brain diseases is poorly characterised. Accordingly, more direct analytical studies are required to identify the therapeutic impact of altering the microbiome on CNS disorders.

Neurological development has been reported to be affected by the microbiota via the microbiota-gut-brain axis, which involves bidirectional communication between host-associated microbes and the central nervous system (CNS). Thus, gut bacteria influences fish behavior and different CNS functions, including brain-derived neurotrophic factor (bdnf) levels and serotonin metabolism. Furthermore, the absence or presence of particular bacterial species in microbiota can significantly modify locomotor and anxiety-like behavior in zebrafish.

In recognition of the potential positive effect that certain bacterial strains may have on the animal gut, various strains have been proposed for use in the treatment of various disease. The following patent documents are cited as example:
EP3072524 and EP3630137 disclose a composition comprising a single *Bacteroides* bacteria species for use in treating psychiatric disorders and a composition comprising a bacterial strain of the genus *Blautia,* for use in a method of treating or preventing a central nervous system disorder However, these documents does not disclose the postbiotic composition of the present invention for use in the treatment of prevention of psychiatric disorders. Furthermore, the postbiotic composition of the invention, contrary to composition disclosed, does not have live microorganisms, which is advantageous as it avoids the possible colonization by these microorganisms as well as the high cost of keeping them viable during the preparation of the composition of the invention.

Also noteworthy are Spanish patent applications P201930242 and P201930280 by the same inventors of the current postbiotic composition describing a human intestinal microbiome modulating composition obtained from lysates of probiotic microorganisms and its method of obtaining as well as a food supplement comprising the composition, which has utility in the prevention and treatment of disorders caused, or at least promoted, by intestinal disbiosis, those documents do not disclose or suggest the use of a composition obtained from lysates of probiotic microorganisms for psychiatric disorders

Accordingly, there is a requirement in the art for new methods of treating central nervous system disorders. Therefore, the present invention aims to solve the problems of the prior art by using an orally administered composition obtained from lysates of probiotic microorganisms for the treatment and/or prevention of psychiatric disorders

### DESCRIPTION OF THE INVENTION

In the present report the expressions "composition of the invention" and "postbiotic composition" are synonymous and are used interchangeably.

In the present report, the term microorganisms include both bacteria and yeast.

Immunomodulatory action is understood, in the context of the present invention, as the set of molecules of the cell walls and lysate of cells and non-viable cells that modulate the innate immune response by the release of cytokines and thereby enhances the immune response against psychiatric disorders.

The terms "treatment" and "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from psychiatric disorders, the terms "treatment" and "treating" refer to the medical management of a subject with the intent to cure, ameliorate, or stabilize psychiatric disorders. In the context of a subject at risk of developing psychiatric disorders, the terms "treatment" and "treating" refer to the medical management of a subject with the intent to prevent psychiatric disorders.

The term "preventing" as used herein refers to administering a compound prior to the onset of clinical symptoms of a disease or conditions so as to prevent a physical manifestation of aberrations associated with the disease or condition. In the context of psychiatric disorders, the term "preventing" refers to administering a compound prior to the onset of clinical symptoms of psychiatric disorders so as to prevent a physical manifestation of aberrations associated with psychiatric disorders.

In a first aspect, the present invention relates to a postbiotic composition that comprises lysates of probiotic microorganisms of the genus *Bacillus, Bifidobacterium, Lactobacillus, Saccharomyces* and *Streptococcus* for use in the treatment and/or prevention of psychiatric disorders in a subject.

In a particular embodiment of the invention, the composition comprises lysates of probiotic microorganisms of *Bacillus licheniformis, Bacillus subtilis, Bacillus mesentericus, Bifidobacterium bifidum, Bifidobacterium breve, Bifidobacterium lactis, Bifidobacterium longum, Lactobacillus acidophilus, Lactobacillus casei, Lactobacillus fermentum, Lactobacillus gasseri, Lactobacillus helveticus, Lactobacillus plantarum, Lactobacillus reuteri, Lactobacillus rhamnosus, Saccharomyces cerevisiae* and *Streptococcus thermophilus* for use in the treatment and/or prevention of psychiatric disorders in a subject..

In another particular embodiment, the present invention relates to a postbiotic composition that comprises lysates of probiotic microorganisms in a percentage amount by weight with respect to the total weight of the lysates of microorganisms of the composition:
- 6% to 20% of bacterial lysates of the genus *Bacillus;*
- 8% to 40% of bacterial lysates of the genus *Bifidobacterium;*
- 8% to 40% of bacterial lysates of the genus *Lactobacillus;*
- 20% to 60% of yeast lysates of the genus *Saccharomyces;*
- 0.5% to 8% of bacterial lysates of the genus *Streptococcus.*
for use in the treatment and/or prevention of psychiatric disorders in a subject.

In a particular embodiment, the amount of bacterial lysates of the genus *Bifidobacterium* and the genus *Lactobacillus* is less than 80% amount by weight with respect to the total weight of the lysates of microorganisms of the composition, preferably, less than 70%, even more preferably less than 65%, even more preferably less than 60%. The amount of bacterial lysates of the genus *Bifidobacterium* and the genus *Lactobacillus* is at least 40%

In accordance with the invention, the composition can comprise between 1% - 99.5% by weight of lysates of microorganisms; particularly 5%-99% by weight of lysates of microorganisms, particularly 10%-96% by weight of lysates of microorganisms, particularly 25%-95% by weight of lysates of microorganisms, or particularly 50%±10% by weight of lysates of microorganisms.

In a particular embodiment, the postbiotic composition of the invention is administered orally or topically.

As indicated previously, the inventors have identified the need to develop a composition that is administered both orally and topically, that is easy to administer and that does not present toxicity to the subject.

Based on this, they have developed a postbiotic composition that can be administered orally or topically, which comprises lysates of microorganisms that can be accompanied by other components and which has proven to be surprisingly beneficial for humans and animals, as it has an immunomodulatory character.

The composition of the invention may comprise additional components or additives or any other substance, such as salts or excipients that facilitate the packaging, preparation and/or administration of the composition but do not affect the capacity of treatment the psychiatric disorders of the postbiotic composition of the invention.

In the context of the present invention, lysates of probiotic microorganisms are understood to be the product obtained after the procedure of culturing and subsequently mechanically or chemically breaking these cells in order to obtain a product with microbial fragments, as well as all the components contained therein. Likewise, this document states that they are lysates of dried probiotic microorganisms, since, thanks to the method of obtaining them, said lysates are subsequently subjected to drying techniques, in such a way that said dried lysates are optionally in the form of a dry powder.

In preferred embodiments of the present invention, the bacterial lysates of the genus *Bacillus* are of one or more of the species selected from the group consisting of: *Bacillus clausii, Bacillus coagulans, Bacillus licheniformis, Bacillus pumilus, Bacillus subtilis, Bacillus mesentericus, Bacillus paralicheniformes,* and combinations thereof, preferably, *Bacillus licheniformis, Bacillus mesentericus* and *Bacillus subtilis.*

In other embodiments, the bacterial lysates of the genus *Bifidobacterium* are of one or more of the species selected from the group consisting of: *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof, preferably *Bifidobacterium bifidum, Bifidobacterium breve, Bifidobacterium lactis and Bifidobacterium longum.*

In other embodiments, the bacterial lysates of the genus *Lactobacillus* are of one or more of the species selected from the group consisting of: *Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus, Lactobacillus salivarius, Lactobacillus helvéticus, Lactobacillus gasseri, Lactobacillus johnsonii, Lactobacillus brevis, Lactobacillus kefiri,* and combinations thereof, preferably *Lactobacillus acidophilus, Lactobacillus casei, Lactobacillus fermentum, Lactobacillus helvéticus, Lactobacillus gasseri Lactobacillus plantarum, Lactobacillus reuteri* and *Lactobacillus rhamnosus.*

In other particular embodiments of the present invention, the lysates of yeasts of the genus *Saccharomyces* are of the species that are selected from the group consisting of *Saccharomyces cerevisiae, Saccharomyces boulardii,* and combinations thereof, preferably *Saccharomyces cerevisiae.*

In other particular embodiments of the present invention, the bacterial lysates of the genus *Streptococcus* are of the species are of the species that are selected from the group consisting of *Streptococcus thermophilus, Streptococcus salivarius,* and combinations thereof, preferably *Streptococcus thermophilus.*

In preferred embodiments of the present invention, the composition of the invention comprises bacterial lysates of the genus *Bacillus* in an amount in percentage by weight between approximately 6 % to 20 % of the total lysates of the composition, preferably 8 % to 17 %, even more preferably between 10% to 15% of the total lysates of the composition. In preferred embodiments, the amount of bacterial lysates of the genus *Bacillus* can be approximately 9%, 10%, 11%, 12%, 13%, 14%, 15% or 16% of the total lysates of the composition.

In a particular embodiment, when the bacterial lysates of the genus *Bacillus* are of the species *Bacillus licheniformis, Bacillus mesentericus* and *Bacillus subtilis,* the percentage by weight of *Bacillus licheniformis* and *Bacillus subtilis* is approximately the same, and is higher than that of *Bacillus mesentericus.*

In preferred embodiments of the present invention, the composition of the invention comprises bacterial lysates of the genus *Bifidobacterium* in an amount in percentage by weight between approximately 8% to 40% of the total lysates of the composition, preferably between 12% to 35%, more preferably between 12% to 30%, even more preferably between 20% to 30% of the total lysates of the composition. In preferred embodiments, the amount of bacterial lysates of the genus *Bifidobacterium* can be approximately 13%, 14%, 15%, 16%, 17%, 18%, 19%, 20%, 21%, 22%, 23%, 24%, 25%, 26%, 27%, 28%, or 29% of the total lysates of the composition.

In a particular embodiment, when the bacterial lysates of the genus *Bifidobacterium* are of the species *Bifidobacterium longum, Bifidobacterium breve, Bifidobacterium bifidum* and *Bifidobacterium lactis,* the percentage by weight of *Bifidobacterium longum* and *Bifidobacterium breve* is approximately the same, and is higher than that of *Bifidobacterium bifidum* and *Bifidobacterium lactis*

In another particular embodiment, the percentage by weight of *Bifidobacterium longum* and *Bifidobacterium breve* is approximately the same, and is higher than that of *Bifidobacterium lactis* which is higher than that of *Bifidobacterium bifidum*

In preferred embodiments of the present invention, the composition of the invention comprises bacterial lysates of the genus *Lactobacillus* in an amount in percentage by weight between approximately 8% to 40% of the total lysates of the composition, preferably between 12% to 35%, more preferably between 12% to 30%, even more preferably between 20% to 30% of the total lysates of the composition. In preferred embodiments, the amount of bacterial lysates of the genus *Lactobacillus* can be approximately 13%, 14%, 15%, 16%, 17%, 18%, 19%, 20%, 21%, 22%, 23%, 24%, 25%, 26%, 27%, 28%, or 29% of the total lysates of the composition.

In a particular embodiment, when the bacterial lysates of the genus *Lactobacillus* are of the species *Lactobacillus acidophilus, Lactobacillus casei, lactobacillus plantarum, Lactobacillus gasseri, Lactobacillus helveticus, Lactobacillus reuteri, Lactobacillus fermentum* and *Lactobacillus rhamnosus,* the percentage by weight of *Lactobacillus plantarum* is smaller than the rest of *Lactobacillus* species: *Lactobacillus acidophilus, Lactobacillus casei, Lactobacillus fermentum, Lactobacillus gasseri, Lactobacillus helvéticus, Lactobacillus plantarum, Lactobacillus reuteri* and *Lactobacillus rhamnosus*

In a particular embodiment, the percentage by weight of *Lactobacillus gasseri,* and *Lactobacillus helveticus* is approximately the same, and is higher than that of *Lactobacillus reuteri* and *Lactobacillus rhamnosus,* which is also approximately the same and is higher than that of *Lactobacillus acidophilus, Lactobacillus casei* and *Lactobacillus fermentum,* which is also approximately the same and is higher than that of *Lactobacillus plantarum.*

In preferred embodiments of the present invention, the composition comprises lysates of yeasts of the genus *Saccharomyces* in an amount in percentage by weight of between approximately 20% to 60% of the total lysates of the composition, preferably 25% to 50% of the total lysates of the composition, preferably between 30% to 45% of the total lysates of the composition. In preferred embodiments, the amount of lysates of yeasts of the genus *Saccharomyces* can be about 31%, 32%, 33%, 34%, 35%,36%, 37%, 38%, 39%, 40%, 41%, 42%, 43% or 44% of the total lysates of the composition. In a particular embodiment, the bacterial lysates of the genus *Saccharomyces* are of *Saccharomyces cereviseae*

In preferred embodiments of the present invention, the composition comprises bacterial lysates of the genus *Streptococcus* in an amount in percentage by weight of between about 0.8% to 8% of the total lysates of the composition, preferably between about 1% to 7%, more preferably between about 2% to 4%, even more preferably between about 1.4% to 3.5% of the total lysates of the composition. In a particular embodiment, the amount of bacterial lysates of the genus *Streptococcus* can be about 1.5%, 1.6%, 1.7%, 1.8%, 1.9%, 2%, 2.1%, 2.2%, 2.3%, 2.4%, 2.5%, 2.6%, 2.7%, 2.8%, 2.9%, 3%, 3.1%, 3.2%, 3.3%, 3.4% or 3.5% of the total lysates of the composition. In other preferred embodiments, the amount of bacterial lysates of the genus *Streptococcus* can be about 3.5%, 4%, 4.5% or 5% of the total lysates of the composition. In a particular embodiment, the bacterial lysates of the genus *Streptococcus* are of *Streptococcus thermophilus.*

In a particular embodiment of the invention, the composition for use, comprises lysates of probiotic microorganisms in an amount in percentage by weight on the total lysates of the composition:
- approximately 8% to 17% of bacterial lysates of the genus *Bacillus;*
- approximately 12% to 35% of bacterial lysates of the genus *Bifidobacterium;*
- approximately 12% to 35% of bacterial lysates of the genus *Lactobacillus;*
- approximately 25% to 50% of yeast lysates of the genus *Saccharomyces;*
- approximately 1% to 7% of bacterial lysates of the genus *Streptococcus.*

This composition is called composition "A".

In another particular embodiment of the invention, the composition for use, comprises lysates of probiotic microorganisms in an amount in percentage by weight on the total lysates of the composition:
- approximately 10% to 15% of bacterial lysates of the genus *Bacillus;*
- approximately 20% to 30% of bacterial lysates of the genus *Bifidobacterium;*
- approximately 20% to 30% of bacterial lysates of the genus *Lactobacillus;*
- approximately 30% to 45% of yeast lysates of the genus *Saccharomyces;*
- approximately 2% to 4% of bacterial lysates of the genus *Streptococcus.*

This composition is called composition "B".

In another particular embodiment of the invention, the composition for use comprises lysates of probiotic microorganisms in an amount in percentage by weight with respect to the total:
- approximately 6% to 20% of bacterial lysates of *Bacillus licheniformis, Bacillus mesentericus* and *Bacillus subtilis;*
- approximately 8% to 40% of bacterial lysates of *Bifidobacterium bifidum, Bifidobacterium breve, Bifidobacterium lactis* and *Bifidobacterium longum,*
- approximately 8% to 40% of bacterial lysates of *Lactobacillus acidophilus, Lactobacillus casei, Lactobacillus fermentum, Lactobacillus helveticus, Lactobacillus gasseri Lactobacillus plantarum, Lactobacillus reuteri* and *Lactobacillus rhamnosus;*
- approximately 20% to 60% of yeast lysates of *Saccharomyces cerevisiae;*
- approximately 0.5% to 8% of bacterial lysates of *Streptococcus thermophilus.*

This composition is called composition "C".

In another particular embodiment of the invention, the composition for use comprises lysates of probiotic microorganisms in an amount in percentage by weight with respect to the total:
- approximately 8% to 17% of bacterial lysates of *Bacillus licheniformis, Bacillus mesentericus* and *Bacillus subtilis;*
- approximately 12% to 35% of bacterial lysates of *Bifidobacterium bifidum, Bifidobacterium breve, Bifidobacterium lactis* and *Bifidobacterium longum,*
- approximately 12% to 35% of bacterial lysates of *Lactobacillus acidophilus, Lactobacillus casei, Lactobacillus fermentum, Lactobacillus helveticus, Lactobacillus gasseri Lactobacillus plantarum, Lactobacillus reuteri* and *Lactobacillus rhamnosus;*
- approximately 25% to 50% of yeast lysates of *Saccharomyces cerevisiae;*
- approximately 1% to 7% of bacterial lysates of *Streptococcus thermophilus.*

This composition is called composition "D".

In another particular embodiment of the invention, the composition for use comprises lysates of probiotic microorganisms in an amount in percentage by weight with respect to the total:
- approximately 10% to 15% of bacterial lysates of *Bacillus licheniformis, Bacillus mesentericus* and *Bacillus subtilis;*
- approximately 20% to 30% of bacterial lysates of *Bifidobacterium bifidum, Bifidobacterium breve, Bifidobacterium lactis* and *Bifidobacterium longum,*
- approximately 20% to 30% of bacterial lysates of *Lactobacillus acidophilus, Lactobacillus casei, Lactobacillus fermentum, Lactobacillus helveticus, Lactobacillus gasseri Lactobacillus plantarum, Lactobacillus reuteri* and *Lactobacillus rhamnosus;*
- approximately 30% to 45% of yeast lysates of *Saccharomyces cerevisiae;*
- approximately 2% to 4% of bacterial lysates of *Streptococcus thermophilus.*

This composition is called composition "E".

In another particular embodiment of the invention, the composition for use comprises lysates of probiotic microorganisms in an amount in percentage by weight with respect to the total:
- approximately 6% to 20% of bacterial lysates of *Bacillus licheniformis, Bacillus mesentericus* and *Bacillus subtilis;* being the percentage by weight of *Bacillus licheniformis* and *Bacillus subtilis* approximately the same, and higher than that of *Bacillus mesentericus.*
- approximately 8% to 40% of bacterial lysates of *Bifidobacterium bifidum, Bifidobacterium breve, Bifidobacterium lactis* and *Bifidobacterium longum,* being the percentage by weight of *Bifidobacterium longum* and *Bifidobacterium breve* approximately the same, and higher than that of *Bifidobacterium lactis* which is higher than that of *Bifidobacterium bifidum*
- approximately 8% to 40% of bacterial lysates of *Lactobacillus acidophilus, Lactobacillus casei, Lactobacillus fermentum, Lactobacillus helveticus, Lactobacillus gasseri Lactobacillus plantarum, Lactobacillus reuteri* and *Lactobacillus rhamnosus;* being the percentage by weight of *Lactobacillus gasseri,* and *Lactobacillus helveticus* approximately the same, and higher than that of *Lactobacillus reuteri* and *Lactobacillus rhamnosus,* which is also approximately the same and is higher than that of *Lactobacillus acidophilus, Lactobacillus casei* and *Lactobacillus fermentum,* which is also approximately the same and is higher than that of *Lactobacillus plantarum.*
- approximately 20% to 60% of yeast lysates of *Saccharomyces cerevisiae;*
- approximately 0.5% to 8% of bacterial lysates of *Streptococcus thermophilus.*

This composition is called composition "F".

In another particular embodiment of the invention, the composition for use comprises lysates of probiotic microorganisms in an amount in percentage by weight with respect to the total:
- approximately 8% to 17% of bacterial lysates of *Bacillus licheniformis, Bacillus mesentericus* and *Bacillus subtilis;* being the percentage by weight of *Bacillus licheniformis* and *Bacillus subtilis* approximately the same, and higher than that of *Bacillus mesentericus.*
- approximately 12% to 35% of bacterial lysates of *Bifidobacterium bifidum, Bifidobacterium breve, Bifidobacterium lactis* and *Bifidobacterium longum,* being the percentage by weight of *Bifidobacterium longum* and *Bifidobacterium breve* approximately the same, and higher than that of *Bifidobacterium lactis* which is higher than that of *Bifidobacterium bifidum*
- approximately 12% to 35% of bacterial lysates of *Lactobacillus acidophilus, Lactobacillus casei, Lactobacillus fermentum, Lactobacillus helveticus, Lactobacillus gasseri Lactobacillus plantarum, Lactobacillus reuteri* and *Lactobacillus rhamnosus;* being the percentage by weight of *Lactobacillus gasseri,* and *Lactobacillus helveticus* approximately the same, and higher than that of *Lactobacillus reuteri* and *Lactobacillus rhamnosus,* which is also approximately the same and is higher than that of *Lactobacillus acidophilus, Lactobacillus casei* and *Lactobacillus fermentum,* which is also approximately the same and is higher than that of *Lactobacillus plantarum.*
- approximately 25% to 50% of yeast lysates of *Saccharomyces cerevisiae;*
- approximately 1% to 7% of bacterial lysates of *Streptococcus thermophilus.*

This composition is called composition "G".

In another particular embodiment of the invention, the composition for use comprises lysates of probiotic microorganisms in an amount in percentage by weight with respect to the total:
- approximately 10% to 15% of bacterial lysates of *Bacillus licheniformis, Bacillus mesentericus* and *Bacillus subtilis;* being the percentage by weight of *Bacillus licheniformis* and *Bacillus subtilis* approximately the same, and higher than that of *Bacillus mesentericus.*
- approximately 20% to 30% of bacterial lysates of *Bifidobacterium bifidum, Bifidobacterium breve, Bifidobacterium lactis* and *Bifidobacterium longum,* being the percentage by weight of *Bifidobacterium longum* and *Bifidobacterium breve* approximately the same, and higher than that of *Bifidobacterium lactis* which is higher than that of *Bifidobacterium bifidum*
- approximately 20% to 30% of bacterial lysates of *Lactobacillus acidophilus, Lactobacillus casei, Lactobacillus fermentum, Lactobacillus helveticus, Lactobacillus gasseri Lactobacillus plantarum, Lactobacillus reuteri* and *Lactobacillus rhamnosus;* being the percentage by weight of *Lactobacillus gasseri,* and *Lactobacillus helveticus* approximately the same, and higher than that of *Lactobacillus reuteri* and *Lactobacillus rhamnosus,* which is also approximately the same and is higher than that of *Lactobacillus acidophilus, Lactobacillus casei* and *Lactobacillus fermentum,* which is also approximately the same and is higher than that of *Lactobacillus plantarum.*
- approximately 30% to 45% of yeast lysates of *Saccharomyces cerevisiae;*
- approximately 2% to 4% of bacterial lysates of *Streptococcus thermophilus.*

This composition is called composition "H".

In other particular embodiments of the present invention, additional components or additives may be: carbohydrates (fructose, xylitol, sorbitol, fructooligosaccharides, inulin), antioxidants (resveratrol, beta-carotene), vitamins (Vitamin C, D, K, B1, B9, E, B3, B5, A, B3 , B6, H, D3, B12 biotin, riboflavin, pyridoxine, pantothenic acid), prebiotics (galacto-oligosaccharides, inulin, oligofructose), amino acids (cysteine, tyrosine, resin, methionine, phenylalanine, glycine, glutamine, alanine, carnitine, glutamine, arginine), lipids (eicosapentaenoic acid, docosahexaenoic acid, arachidonic acid), trace elements (sodium, potassium, magnesium, phosphorus, calcium, copper, zinc, manganese, chromium, iodine, selenium), digestive enzymes (papain, amylase, lactase, bromelain), whey.

These micro and macronutrients can modulate the intestinal microbiota and favor the predominance of some species associated with a good state of health, such as species of the genera *Bifidobacterium, Lactobacillus, Akkermansia, Fecalibacterium, Eubacterium, Roseburia, Ruminococcus* and *Blautia.* Among the macronutrients, carbohydrates such as galactooligosaccharides, fructooligosaccharides and inulin have a bifidogenic effect and can modulate microorganisms beneficial to health as well as unsaturated fat, which also increases the beneficial microbiota. Antioxidants such as polyphenols modulate the Firmicutes/Bacteroidetes ratio. Vitamins, such as vitamin A, C, D and E have a positive influence on *Bifidobacteria, Akkermansia* and *Lactobacillus.* Some trace elements such as calcium, magnesium, phosphorus, selenium and zinc have shown an effect on *Akkermansia, Bifidobacterium* and *Ruminococcus.* Prebiotic consumption has been associated with the growth of *Bifidobacterium* and lactic acid bacteria. In general, macro and micronutrients influence the composition and diversity of the gut microbiota.

In a particular embodiment of the invention, the composition of lysates and additional components is that of table 1. Additional components may be one or more of resveratrol, astaxanthin, bromelain, papain, fermented rice starch, corn starch and FOS, each of these components in varying amounts, as shown in Table 1.

**Table 1: List of additional components to the composition of the invention per 100 mg of lysates of probiotic microorganisms.**

| | | |
|---|---|---|
| Composition of lysates of probiotic microorganisms | 100 | Mg |
| Resveratrol | Up to 2.55 | Mg |
| Astaxanthin | Up to 25 | Mg |
| Bromelain | Up to 24 | Mg |
| Papain | Up to 35.2 | Mg |
| Fermented rice starch | Up to 110 | Mg |
| Corn starch | Up to 12 | Mg |
| FOS | Up to 70 | Mg |

In the specific example of Table 1, the composition of lysates of probiotic microorganisms would represent 26.4% of the total weight of the composition of the invention.

Another object of the invention relates to a method to obtained the postbiotic composition described above which comprised the following steps:
- Cultivate the microbial species selected for the preparation of the composition using the standard conditions established for the microbial species listed in this document;
- Filter or centrifuge the microbial species until a suitable biomass of the selected microorganisms is obtained;
- Lyse through freezing and thawing and sonication cycles of the biomasses obtained. until securing at least 90% or 91% or 92% or 94% of bacterial lysates, preferably at least 95%, or 96% or 97% or 98% and more preferably, 99% or 100%;
- Mix the different strains in the proportions selected according to the percentage by weight to obtain the composition of lysates of probiotic microorganisms of the invention.

A skilled person would know how to find the appropriate protocol for the growth of the microorganisms of the composition, as well as the protocol for their lysis, since these are common procedures in the prior art.

In a particular embodiment, the method of obtaining the composition of the invention comprises an additional step: drying by atomization or lyophilization the biomasses of bacterial lysates obtaining a product in powder form comprising all the bacterial strains selected to prepare the composition object of the invention.

In a particular embodiment, the composition of the invention is presented in the form of dry powder.

When the composition of the invention is presented in the form of dry powder, it is necessary to dissolve it in water or any other liquid that does not affect the properties of the components of the composition prior to its intake by the patient. The advantages of dry powder are that it is easier to transport and to store, and it also increases the stability and durability of the composition. In the present document, dry powder means that the humidity level is less than 10%, preferably less than 5%, even more preferably less than 1%.

In particular embodiments, the composition of the invention is obtained from cultures of probiotic microorganisms of the genera described in the present document, which comprise a certain amount of Colony Forming Units (CFU). In the context of the present invention, a Colony Forming Unit is a term of microbiology. It is an indicator of the amount of live microorganisms present in a medium.

The process of obtaining lysates comprises a thermal treatment, alternating heating and cooling cycles. Each batch of viable cell culture is resuspended in distilled water in a 1:2 ratio and subjected to a sterilization cycle in an autoclave at 121°C for 20 minutes. They are then frozen at -20°C for 12 hours and thawed at room temperature the next day.

Once thawed, they undergo a cell rupture treatment by sonication for 20min, at an output power of 500 W at an amplitude of 40% and 10 seconds of pause, obtaining a mass of lysed cells from the probiotic batch (Qsonica, Q500). The final solution is freeze-dried and ground to obtain a lysate of the powdery probiotic microorganism. The powder is kept in a cool environment away from heat. The degree of survival in no case exceeds a final viability of approximately 7E+3 CFU/ml, which implies practically 100% cell death.

In particular embodiments of the present invention, one starts with a quantity of bacteria of the genus *Bacillus* comprising approximately between 3.49% to 20.94% of CFU with respect to the total CFU of the composition, preferably between approximately 9.98% and 15.75%. In absolute values, the culture grown, before proceeding to the lysis of the cells, presents between 1E+11 and 5E+11 cells, preferably between 2E+11 and 3E+11, most preferably approximately 2.30E+11.

In another particular embodiment, when the species of the genus *Bacillus* are: *Bacillus licheniformis, Bacillus mesentericus* and *Bacillus subtilis,* each of the grown cultures has, before proceeding to the lysis of the cells, between 6E+10 and 5E+11 of *Bacillus licheniformis* cells, between 7E+10 and 8E+10 of *Bacillus mesentericus* cells and between 7E+10 and 5E+11 of *Bacillus subtilis* cells.

In particular embodiments of the present invention, one starts with a quantity of bacteria of the genus *Bifidobacterium* comprising between 5.20% to 33.64% of CFU with respect to the total culture, preferably between 8.99% to 20.75%. In absolute values, the culture grown, before proceeding to the lysis of the cells, presents between 8E+10 and 7E+11 cells, preferably between 1E+11 and 4E+11, most preferably approximately 2.70E+11.

In another particular embodiment, when the species of the genus *Bifidobacterium* are: *Bifidobacterium bifidum, Bifidobacterium breve, Bifidobacterium lactis* and *Bifidobacterium longum,* each of the grown cultures has, before proceeding to the lysis of the cells, between 8E+10 and 5E+11 of *Bifidobacterium bifidum* cells, between 7E+9 and 6E+10 of *Bifidobacterium breve* cells, between 8E+10 and 5E+11 of *Bifidobacterium lactis* cells and between 7E+9 and 6E+10 of *Bifidobacterium longum* cells.

In particular embodiments of the present invention, one starts with a number of bacteria of the genus *Lactobacillus* comprising between 22.64% to 66.82% of CFU with respect to the total CFU of the composition, preferably between 30.66% to 57.36%. In absolute values, the culture grown, before proceeding to the lysis of the cells, presents between 2E+11 and 1E+12 cells, preferably between 5E+11 and 9E+11, most preferably approximately 7.5E+11.

In another particular embodiment, when the species of the genus *Lactobacillus* are: *Lactobacillus acidophilus, Lactobacillus casei, Lactobacillus fermentum, Lactobacillus gasseri, Lactobacillus helveticus, Lactobacillus plantarum, Lactobacillus reuteri,* and *Lactobacillus rhamnosus,* each of the grown cultures presents, before proceeding to the lysis of the cells, between 1E+10 and 7E+10 cells of *Lactobacillus acidophilus,* between 8E+10 and 4E+11 cells of *Lactobacillus casei,* between 1E+10 and 7E+10 cells of *Lactobacillus fermentum,* between 2E+9 and 9E+9 cells of *Lactobacillus gasseri,* between 2E+9 and 9E+9 cells of *Lactobacillus helveticus,* between 7E+10 and 5E+11 cells of *Lactobacillus plantarum,* between 2E+9 and 9E+9 cells of *Lactobacillus reuteri* and between 1E+11 and 6E+11 cells of *Lactobacillus rhamnosus.*

In particular embodiments of the present invention, one starts with a quantity of bacteria of the genus *Saccharomyces* comprising between 11.44% to 24.79% of CFU with respect to the total culture, preferably between 16.04% to 22.75%. In absolute values, the culture grown, before proceeding to the lysis of the cells, presents between 9E+10 and 7E+11 cells, preferably between 2E+11 and 6E+11, most preferably approximately 3,80E+11. In a particular embodiment, the species of *Saccharomyces* is *Saccharomyces cerevisiae.*

In particular embodiments of the present invention, one starts with a number of bacteria of the genus *Streptococcus* comprising between 5.72% to 38.15% of CFU with respect to the total CFU of the composition, preferably between 10.98% to 20.75%. In absolute values, the culture grown, before proceeding to the lysis of the cells, presents between 1E+11 and 6E+11 cells, preferably between 2E+11 and 4E+11, most preferably approximately 3.00E+11. In a particular embodiment, the species of *Streptococcus* is *Streptococcus thermophilus.*

These probiotic bacteria are cultured under standard conditions, as set out in the culture protocols published by the Spanish Collection of Type Cultures (CECT), indicated for each of the bacterial species described in this document.

Once these microorganisms are cultured, they are subjected to a lysis procedure. First of all, the microbial cells undergo heat treatment. Each batch of viable cell culture undergoes a sterilization cycle in an autoclave at 121°C for 20 to 30 minutes. This temperature denatures and coagulates the proteins by inactivating them. In addition, it causes damage to membranes, aggregation of ribosomes, breaking of DNA strands and inactivation of enzymes. Once cold, they undergo a cell rupture treatment by sonication for a period of 15 to 20min, at an output power of between 450 to 550 W, at an amplitude of between 38 to 43% and a period of 8 to 15 seconds of pause, obtaining a mass of lysed cells from the probiotic batch (Qsonica, Q500). This achieves the breakage of intermolecular interactions and DNA fragmentation. Optionally, the final solution is freeze-dried and ground to obtain a lysate of the powdery probiotic microorganism. The powder is kept in a cool environment away from heat.

The composition, in accordance with the invention, is the result of the combination of probiotic microorganisms that, after a process of growth and lysis, generates an extract consisting of a set of metabolites, proteins, DNA fragments and other components, such as, for example, peptidoglycans, which through efficient dosing is able to alter and modify in a surprising way the microbiota of the host through several mechanisms in such a way that it activates the immune system, reversing dysbiosis and surprisingly helping to improve psychiatric disorders.

The technical effect deriving from the composition of the invention is that it acts directly on the microbiota of a subject. Thanks to this restorative and modulating action, the inventors have confirmed that, surprisingly, intervening on the microbiota of a subject can significantly improve the health state of said subject, and in particular the condition of psychiatric disorders, as demonstrated by the experimental results described in the present application.

In a particular embodiment, when the composition of the invention is in powder form, the composition, in line with the first aspect of the invention, can be presented in sealed sachets, which is in itself conventional in the food and pharmaceutical industry.

In the context of the present invention, a dose is defined as the amount of the composition of the invention that is to be effective, efficacious and safe for the subject and to solve the health problem for which it has been indicated.

Thus, in the context of the present invention, it is established that at least one dose must be administered, preferably at least one daily dose, between 100mg to 80000mg of the composition of the invention, including any of the compositions A-H. In preferred embodiments, a dosage may comprise between 100mg to 50000mg of the composition of the invention, including any of the compositions A-H; in another preferred embodiment, a dosage may comprise between 100mg to 2000mg of the composition of the invention, including any of the compositions A-H; in another preferred embodiment, a dosage may comprise between 100mg to 700mg of the composition of the invention, including any of the compositions A-H. In particular embodiments of the present invention, a dosage may comprise approximately 120mg, 130mg, 140mg, 150mg, 160mg, 170mg, 180mg, 190mg, 200mg 210mg, 220mg, 230mg, 240mg, 250mg, 260mg, 270mg, 280mg, 290mg, 300mg 310mg, 320mg, 330mg, 340mg, 350mg, 360mg, 370mg, 380mg, 390mg, 400mg, 410mg, 420mg, 430mg, 440mg, 450mg, 460mg, 470mg, 480mg, 490mg, 500mg, 510mg, 520mg, 530mg, 540mg, 550mg, 560mg, 570mg, 580mg, 590mg, 600mg, 610mg, 620mg, 630mg, 640mg, 650mg, 660mg, 670mg, 680mg, or 690mg of the composition of the invention, including any of the compositions A-H.

In a particular embodiment, you can administer at least one dose of the composition of the invention, including any of the compositions A-H, daily, or every other day, or every 3 or 4 days, following any of the concentrations of the preceding paragraph.

In a particular embodiment the dosage is at least 100 mg/day, or at least 100 md/day, or between 80 and 150 mg/day, or between 80 and 200 mg/day.

In another additional embodiment, the administration of the postbiotic composition of the invention, including any of the compositions A-H, is daily for at least 2 weeks, preferably daily for at least 3 weeks, even more preferably daily for at least 4 weeks, even more preferably daily for at least 5 weeks, even more preferably daily for at least 6 weeks, even more preferably daily for at least 7 weeks, even more preferably daily for at least 8 weeks, or daily for at least 9 weeks, or daily for at least 10 weeks, or daily for at least 11 weeks, or daily for at least 12 weeks.

Preferably the composition of the invention, including any of the compositions A-H, is administered in an amount between 100mg to 700mg per dose and between 1 to 6 doses per day for at least 2 weeks.

In another additional embodiment, the treatment with the postbiotic composition is carried out before, simultaneously or after the treatment of the patient with any other psychiatric treatment. When the treatment with the composition of the invention manages to improve the general condition of the patient, any other psychiatric treatment can be performed if the doctors consider that the patient can withstand them.

An additional object of the invention relates to a pharmaceutical formulation comprising an effective pharmaceutical amount of the composition, in accordance with the first aspect of the invention, and at least one pharmaceutically acceptable excipient for use in the treatment of psychiatric disorders.

In the context of the present invention, the expression "pharmaceutical composition" refers to a formulation that has been adapted to deliver a predetermined dose of one or more useful therapeutic agents to a cell, a group of cells, an organ, or a tissue.

The expression "effective pharmaceutical quantity", as used herein, is understood as a nontoxic amount, capable of providing a therapeutic effect. The exact amount required will vary from one subject to another, depending on the species, age and general condition of the subject, the severity of the disease being treated, the particular compound used, its mode of administration, and the like. Therefore, it is not possible to specify an exact "effective quantity". However, an appropriate effective amount can be determined by a skilled person through routine experimentation. In the context of compounds and compositions for the prevention of infection, the pharmaceutically effective quantity can refer to the amount necessary to relieve the symptoms of psychiatric disorders suffered by the subject and improve their quality of life.

Also, in the context of the present invention, "pharmaceutically acceptable excipient" means a therapeutically inactive substance that is said to be used to incorporate the active ingredient, and that is acceptable to the patient from a pharmacological/toxicological point of view and to the pharmaceutical chemist who manufactures it from a physical/chemical point of view, with respect to composition, formulation, stability, patient acceptance and bioavailability.

The pharmaceutical formulation of the invention can be suited for topical administration or oral administration. This is, the at least one pharmaceutical acceptable excipient is suitable for topical or oral administration.

A topical administration is that that is applied directly over the skin or the mucosa.

Formulations for topical administration can include ointments, lotions, creams, gels, drops, suppositories, sprays, serums, oils liquids, and powders. Conventional pharmaceutical carriers, aqueous, powder or oily bases, thickeners and the like can be necessary or desirable.

In a particular embodiment, the formulation is administered as a cream. It may contain anti-inflammatories such as aloe vera, skin regenerators such as vitamin D, antioxidants such as vitamin C, moisturisers such as glycerine, collagen stimulators such as glycolic acid, and anti-ageing ingredients such as peptides, retinol and hyaluronic acid. For example, the ingredients that can be used are: water, Glycerin, Butyrospermun Parkii Butter, Cetyl Palmitate, Dimethicone, Sodium, Polyacrylate, Phenoxyethanol, linalool, Ctitronellol, alpha-isomethyl Ionone, geraniol, mentol, parfum, Vitamin D, Aloe Vera, Vitamin E, Almond oil.

Formulations for oral administration can include powders or granules, suspensions or solutions in water or non-aqueous media, capsules, sachets, or tablets. Thickeners, flavorings, diluents, emulsifiers, dispersing aids or binders can be desirable.

Another additional object of the invention relates to a food supplement comprising the composition of the invention for use in the treatment of a patient with an psychiatric disorder, including any of the compositions "A-H".

In the context of the present invention, what is understood by food supplement is a food product whose purpose it is to supplement the normal diet, and which consists of concentrated sources of nutrients or other substances, that has a nutritional or physiological effect, in simple or combined form, and is marketed in dosed form, ie capsules, tablets, pills and other similar means, sachets of powders, ampoules of liquid, dropper bottles, and other similar forms of liquids and powders to be taken in small unit quantities, as defined in Directive 2002/46/EC of the European Parliament.

In a particular embodiment, the food supplement, in accordance with the invention, is in capsule form, such as conventional gelatin capsules, inside which the composition in powder form is contained.

In particular embodiments where the composition is presented in sealed sachets, said composition can be administered to the patient dissolved or suspended in a liquid, preferably in an aqueous liquid, and more preferably, in beverages such as fruit juices, milk, water. In addition, it can also be mixed with foods such as yoghurt, liquid yoghurt, soups, purees, creams, or porridges. These foods must be at optimum temperature to be consumed, and never heated after having added the composition of the invention.

As it will be shown later, the composition of the invention is effective as a food supplement orally administered.

The postbiotic composition of the invention being composed of lysates of microorganisms of multiple probiotic species and/or the formulation comprising said composition, is intended to prevent and/or treat, this is, to control or at least mitigate psychiatric disorders in a subject, particularly those psychiatric disorders related to inflammatory diseases and/or oxidative stress, more concretely to restore alterations that lead to dysbiosis and have an impact on oxidative and inflammatory processes. In addition, contrary to compositions comprising probiotics, it avoids the possible colonization by these microorganisms as well as the high cost of keeping them viable during the preparation of the composition of the invention.

In a particular embodiment, the psychiatric disorder is selected from the group consisting of: (i) an obsessive compulsive disorder, (ii) a depressive disorder, (iii) a schizophrenia disorder, (iv) a schizotypal disorder, (v) an anxiety disorder, (vi) substance abuse, (vii) and/or an avolition disorder, preferably an anxiety disorder.

As used herein the term 'psychiatric disorder' is a clinically significant behavioural or psychological syndrome or pattern that occurs in an individual and that is associated with present distress (e.g., a painful symptom) or disability (i.e., impairment in one or more important areas of functioning) or with a significantly increased risk of suffering death, pain, disability, or an important loss of freedom.

Diagnostic criteria for psychiatric and neurological disorders referred to herein are provided in, for example, the Diagnostic and Statistical Manual of Mental Disorders, Fifth Edition, (DSM-5).

As used herein the term 'obsessive-compulsive disorder' is defined by the presence of either obsessions or compulsions, but commonly both. The symptoms can cause significant functional impairment and/or distress. An obsession is defined as an unwanted intrusive thought, image or urge that repeatedly enters the person's mind. Compulsions are repetitive behaviours or mental acts that the person feels driven to perform. Typically obsessive-compulsive disorder (OCD) manifests as one or more obsession which drives adoption of a compulsion. For example, an obsession with germs may drive a compulsion to clean. A compulsion can either be overt and observable by others, such as checking that a door is locked, or a covert mental act that cannot be observed, such as repeating a certain phrase in one's mind.

As used herein the term 'depressive disorder' includes major depressive disorder, persistent depressive disorder, bipolar disorder, bipolar depression, and depression in terminally ill patients.

As used herein the term 'major depressive disorder' (MDD, also referred to as major depression or clinical depression) is defined as the presence of five or more of the following symptoms over a period of two-weeks or more (also referred to herein as a 'major depressive episode'), most of the day, nearly every day:
- depressed mood, such as feeling sad, empty or tearful (in children and teens, depressed mood can appear as constant irritability);
- significantly reduced interest or feeling no pleasure in all or most activities;
- significant weight loss when not dieting, weight gain, or decrease or increase in appetite (in children, failure to gain weight as expected);
- insomnia or increased desire to sleep;
- either restlessness or slowed behaviour that can be observed by others;
- fatigue or loss of energy;
- feelings of worthlessness, or excessive or inappropriate guilt;
- trouble making decisions, or trouble thinking or concentrating;
- recurrent thoughts of death or suicide, or a suicide attempt.

At least one of the symptoms must be either a depressed mood or a loss of interest or pleasure.

Persistent depressive disorder, also known as dysthymia, is defined as a patient exhibiting the following two features:
A. has depressed mood for most the time almost every day for at least two years. Children and adolescents may have irritable mood, and the time frame is at least one year.
B. While depressed, a person experiences at least two of the following symptoms:
   - Either overeating or lack of appetite.
   - Sleeping too much or having difficulty sleeping.
   - Fatigue, lack of energy.
   - Poor self-esteem.
   - Difficulty with concentration or decision making.

As used herein the term 'treatment resistant depression' describes MDD which fails to achieve an adequate response to an adequate treatment with standard of care therapy.

As used herein 'bipolar disorder' also known as manic-depressive illness, is a disorder that causes unusual shifts in mood, energy, activity levels, and the ability to carry out day-to-day tasks.

There are two defined sub-categories of bipolar disorder; all of them involve clear changes in mood, energy, and activity levels. These moods range from periods of extremely "up," elated, and energized behaviour (known as manic episodes, and defined further below) to very sad, "down," or hopeless periods (known as depressive episodes). Less severe manic periods are known as hypomanic episodes.

Bipolar I Disorder- defined by manic episodes that last at least 7 days, or by manic symptoms that are so severe that the person needs immediate hospital care. Usually, depressive episodes occur as well, typically lasting at least 2 weeks. Episodes of depression with mixed features (having depression and manic symptoms at the same time) are also possible.

Bipolar II Disorder- defined by a pattern of depressive episodes and hypomanic episodes, but not the full-blown manic episodes described above.

As used herein 'bipolar depression' is defined as an individual who is experiencing depressive symptoms with a previous or coexisting episode of manic symptoms, but does not fit the clinical criteria for bipolar disorder.

As used herein the term 'anxiety disorder' includes generalised anxiety disorder, phobia, panic disorder, social anxiety disorder, and post-traumatic stress disorder.

'Generalised anxiety disorder' (GAD) as used herein means a chronic disorder characterised by long-lasting anxiety that is not focused on any one object or situation. Those suffering from GAD experience non-specific persistent fear and worry, and become overly concerned with everyday matters. GAD is characterised by chronic excessive worry accompanied by three or more of the following symptoms: restlessness, fatigue, concentration problems, irritability, muscle tension, and sleep disturbance.

'Phobia' is defined as a persistent fear of an object or situation the affected person will go to great lengths to avoid, typically disproportional to the actual danger posed. If the feared object or situation cannot be avoided entirely, the affected person will endure it with marked distress and significant interference in social or occupational activities.

A patient suffering from a 'panic disorder' is defined as one who experiences one or more brief attack (also referred to as a panic attack) of intense terror and apprehension, often marked by trembling, shaking, confusion, dizziness, nausea, and/or difficulty breathing. A panic attack is defined as a fear or discomfort that abruptly arises and peaks in less than ten minutes.

'Social anxiety disorder' is defined as an intense fear and avoidance of negative public scrutiny, public embarrassment, humiliation, or social interaction. Social anxiety often manifests specific physical symptoms, including blushing, sweating, and difficulty speaking.

'Post-traumatic stress disorder' (PTSD) is an anxiety disorder that results from a traumatic experience. Post-traumatic stress can result from an extreme situation, such as combat, natural disaster, rape, hostage situations, child abuse, bullying, or even a serious accident. Common symptoms include hypervigilance, flashbacks, avoidant behaviours, anxiety, anger and depression.

As used herein the term 'substance abuse' means a patterned use of a drug in which the user consumes the substance in amounts or with methods which are harmful to themselves or others.

As used herein the term 'an avolition disorder' refers to a disorder which includes as a symptom the decrease in motivation to initiate and perform self-directed purposeful activities.

As used herein, "subject" or "patient" includes mammals (e.g., a human, horse, pig, rabbit, dog, sheep, goat, non-human primate, cow, cat, guinea pig or rodent), a fish, a bird or a reptile or an amphibian. The subject can be an invertebrate, more specifically an arthropod (e.g., insects and crustaceans). The term does not denote a particular age or sex. Thus, adult and newborn subjects, as well as fetuses, whether male or female, are intended to be covered. A patient refers to a subject afflicted with a disease or disorder. The term "patient" includes human and veterinary subjects. In one aspect, the compounds described herein can be administered to a subject comprising a human or an animal including, but not limited to, a mouse, dog, cat, horse, bovine or ovine and the like, that is in need of alleviation or amelioration from a recognized medical condition.

Therefore, the main advantages deriving from the postbiotic composition of the invention are the following:
- the physiological functions of the microbiota of humans and animals are supplemented and/or complemented;
- the alterations that lead to dysbiosis and have an impact on oxidative and inflammatory processes are restored;
- the fact that the product is administered topically, makes the present composition, and its surprising effect, a very advantageous alternative to current psychiatric treatments;
- the present composition comprises lysates of microorganisms, i.e. it does not contain living organisms, which makes the present composition a safe alternative, because it avoids the possible dangers of colonising organisms, and it does not have the toxicity that a conventional drug can have.

Additionally, the invention comprises the following clauses:
1. A postbiotic composition that comprises lysates of probiotic microorganisms of the genus *Bacillus, Bifidobacterium, Lactobacillus, Saccharomyces* and *Streptococcus* for use in the treatment and/or prevention of psychiatric disorders in a subject.
2. The postbiotic composition for use, according to the preceding clause, wherein the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus Bacillus paralicheniformes,* and combinations thereof, preferably *Bacillus licheniformis, Bacillus mesentericus* and *Bacillus subtilis.*
3. The postbiotic composition for use, according to any of the preceding clauses, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof, preferably *Bifidobacterium bifidum, Bifidobacterium breve, Bifidobacterium lactis and Bifidobacterium longum.*
4. The postbiotic composition for use, according to any of the preceding clauses, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof, preferably *Lactobacillus acidophilus, Lactobacillus casei, Lactobacillus fermentum, Lactobacillus helvéticus, Lactobacillus gasseri Lactobacillus plantarum, Lactobacillus reuteri* and *Lactobacillus rhamnosus.*
5. The postbiotic composition for use, according to any of the preceding clauses, wherein the lysates of yeast genus *Saccharomyces* are of the species that are selected from the group consisting of *Saccharomyces cerevisiae, Saccharomyces boulardii,* and combinations thereof, preferably *Saccharomyces cerevisiae.*
6. The postbiotic composition for use, according to any of the preceding clauses, wherein the bacterial lysates of the genus *Streptococcus* are of the species that are selected from the group consisting of *Streptococcus thermophilus, Streptococcus salivarius,* and combinations thereof, preferably *Streptococcus thermophilus.*
7. The postbiotic composition for use, according to the preceding clauses 1 to 6 wherein the composition comprises lysates of probiotic microorganisms of *Bacillus licheniformis, Bacillus subtilis, Bacillus mesentericus, Bifidobacterium bifidum, Bifidobacterium breve, Bifidobacterium lactis, Bifidobacterium longum, Lactobacillus acidophilus, Lactobacillus casei, Lactobacillus fermentum, Lactobacillus gasseri, Lactobacillus helveticus, Lactobacillus plantarum, Lactobacillus reuteri, Lactobacillus rhamnosus, Saccharomyces cerevisiae* and *Streptococcus thermophilus*
8. The postbiotic composition for use, according to the preceding clause, wherein the percentage by weight of *Bacillus licheniformis* and *Bacillus subtilis* is approximately the same, and is higher than that of *Bacillus mesentericus.*
9. The postbiotic composition for use, according to any of the preceding clauses 7 or 8, wherein the percentage by weight of *Bifidobacterium longum* and *Bifidobacterium breve* is approximately the same, and is higher than that of *Bifidobacterium lactis* which is higher than that of *Bifidobacterium bifidum.*
10. The postbiotic composition for use, according to any of the preceding clauses 7 to 9, wherein the percentage by weight of *Lactobacillus gasseri,* and *Lactobacillus helveticus* is approximately the same, and is higher than that of *Lactobacillus reuteri* and *Lactobacillus rhamnosus,* which is also approximately the same and is higher than that of *Lactobacillus acidophilus, Lactobacillus casei* and *Lactobacillus fermentum,* which is also approximately the same and is higher than that of *Lactobacillus plantarum.*
11. The postbiotic composition for use, according to any of the preceding clauses, that comprises lysates of probiotic microorganisms in a percentage amount by weight with respect to the total weight of the lysates of microorganisms of the composition:
   - 6% to 20% of bacterial lysates of the genus *Bacillus;*
   - 8% to 40% of bacterial lysates of the genus *Bifidobacterium;*
   - 8% to 40% of bacterial lysates of the genus *Lactobacillus;*
   - 20% to 60% of yeast lysates of the genus *Saccharomyces;*
   - 0.5% to 8% of bacterial lysates of the genus *Streptococcus.*
12. The composition for use, according to the preceding clause, wherein the amount of bacterial lysates of the genus *Bifidobacterium* and the genus *Lactobacillus* is less than 80% amount by weight with respect to the total weight of the lysates of microorganisms of the composition
13. The composition for use, according to any of the preceding clauses, that is administered orally or topically.
14. The composition for use, according to any of the preceding clauses, that comprises between 1% and 99.5% by weight of lysates of microorganisms, particularly 5%-99% by weight of lysates of microorganisms, particularly 10%-96% by weight of lysates of microorganisms, 25%-95% by weight of lysates of microorganisms, or particularly 50%+10% by weight of lysates of microorganisms.
15. The composition for use, according to any of the preceding clauses, wherein the bacterial lysates of the genus *Bacillus* are approximately 8% to 17% of the total lysates of the composition.
16. The composition for use, according to any of the preceding clauses, wherein the percentage by weight of *Bacillus licheniformis* and *Bacillus subtilis* is approximately the same, and is higher than that of *Bacillus mesentericus.*
17. The composition for use, according to any of the preceding clauses, wherein bacterial lysates of the genus *Bifidobacterium* are approximately 12% to 35% of the total lysates of the composition.
18. The composition for use, according to any of the preceding clauses, wherein the percentage by weight of *Bifidobacterium longum* and *Bifidobacterium breve* is approximately the same, and is higher than that of *Bifidobacterium bifidum* and *Bifidobacterium lactis,* or the percentage by weight of *Bifidobacterium longum* and *Bifidobacterium breve* is approximately the same, and is higher than that of *Bifidobacterium lactis* which is higher than that of *Bifidobacterium bifidum*
19. The composition for use, according to any of the preceding clauses, wherein bacterial lysates of the genus *Lactobacillus* are about 12% to 35% of the total lysates of the composition.
20. The composition for use, according to any of the preceding clauses, wherein the percentage by weight of *Lactobacillus plantarum* is smaller than the rest of *Lactobacillus* species: *Lactobacillus acidophilus, Lactobacillus casei, Lactobacillus fermentum, Lactobacillus gasseri, Lactobacillus helveticus, Lactobacillus plantarum, Lactobacillus reuteri* and *Lactobacillus rhamnosus,* or the percentage by weight of *Lactobacillus gasseri,* and *Lactobacillus helveticus* is approximately the same, and is higher than that of *Lactobacillus reuteri* and *Lactobacillus rhamnosus,* which is also approximately the same and is higher than that of *Lactobacillus acidophilus, Lactobacillus casei* and *Lactobacillus fermentum,* which is also approximately the same and is higher than that of *Lactobacillus plantarum*
21. The composition for use, according to any of the preceding clauses, wherein the lysates of yeast of the genus *Saccharomyces* are approximately between 25% to 50% of the total lysates of the composition.
22. The composition for use, according to any of the preceding clauses, wherein the lysates of yeast of the genus *Streptococcus* are approximately between 1% to 7% of the total lysates of the composition.
23. The composition for use, according to any of the preceding clauses, wherein the composition comprises lysates of probiotic microorganisms in an amount in percentage by weight of the total lysates of the composition according to composition A.
24. The composition for use, according to any of the preceding clauses, wherein the composition comprises lysates of probiotic microorganisms in an amount in percentage by weight of the total lysates of the composition according to composition B.
25. The composition for use, according to any of the preceding clauses, wherein the composition comprises lysates of probiotic microorganisms in an amount in percentage by weight of the total lysates of the composition according to composition C.
26. The composition for use, according to any of the preceding clauses, wherein the composition comprises lysates of probiotic microorganisms in an amount in percentage by weight of the total lysates of the composition according to composition D.
27. The composition for use, according to any of the preceding clauses, wherein the composition comprises lysates of probiotic microorganisms in an amount in percentage by weight of the total lysates of the composition according to composition E.
28. The composition for use, according to any of the preceding clauses, wherein the composition comprises lysates of probiotic microorganisms in an amount in percentage by weight of the total lysates of the composition according to composition F.
29. The composition for use, according to any of the preceding clauses, wherein the composition comprises lysates of probiotic microorganisms in an amount in percentage by weight of the total lysates of the composition according to composition G.
30. The composition for use, according to any of the preceding clauses, wherein the composition comprises lysates of probiotic microorganisms in an amount in percentage by weight of the total lysates of the composition according to composition H.
31. A procedure for obtaining a postbiotic composition for use in the treatment of skin disorders, according to any of the clauses 1 to 30 comprising:
   - Cultivating the selected microbial species
   - Filter or centrifuge the cultures until an adequate biomass of the selected microorganisms is obtained
   - Lysis through cycles of freezing and thawing, and sonication of the obtained the biomasses
32. The process, according to the preceding clause, comprising drying by atomization or lyophilization of the biomasses of bacterial lysates obtaining a product in the form of dry powder.
33. A pharmaceutical formulation comprising an effective pharmaceutical quantity of the postbiotic composition, according to any of the preceding clauses 1 to 30, and at least one pharmaceutically acceptable excipient for use in the treatment of psychiatric disorders.
34. The pharmaceutical formulation for use, according to the preceding clause, wherein the at least one pharmaceutical acceptable excipient is suitable for topical or oral administration.
35. The pharmaceutical formulation for use, according to any of the preceding clauses 33 to 34, wherein formulations for topical administration are ointments, lotions, creams, gels, drops, suppositories, sprays, serums, oils liquids, and powders.
36. The pharmaceutical formulation for use, according to any of the preceding clauses 34 to 35, wherein the formulation is administered as a cream and comprises anti-inflammatories, antioxidants, moisturisers, collagen stimulators, and anti-ageing ingredients.
37. The pharmaceutical formulation for use, according to any of the preceding clauses 33 to 34, wherein formulations for oral administration are powders or granules, suspensions or solutions in water or non-aqueous media, capsules, sachets, or tablets
38. A food supplement comprising the postbiotic composition, according to any of the clauses 1 to 30, for use in the treatment of psychiatric disorders in a subject.
39. The composition for use, according to any of the preceding clauses 1 to 30, the pharmaceutical formulation for use, according to any of the preceding clauses 33 to 37 and/or the food supplement for use, according to clause 38, wherein the psychiatric disorder is selected from the group consisting of: an obsessive compulsive disorder, a depressive disorder, a schizophrenia disorder, a schizotypal disorder, an anxiety disorder, substance abuse, and/or an avolition disorder, preferably an anxiety disorder.
40. The composition for use, the pharmaceutical formulation for use, and/or the food supplement for use, according to the preceding clause, wherein anxiety disorder comprises generalised anxiety disorder, phobia, panic disorder, social anxiety disorder, and post-traumatic stress disorder.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "approximately" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1 , about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below

### BRIEF DESCRIPTION OF THE FIGURES

To help a better understanding of the invention, a set of figures is included as an integral part of this description, where, for illustrative and non-limiting purposes, graphs of the experimental results obtained in the following examples are presented in this document.

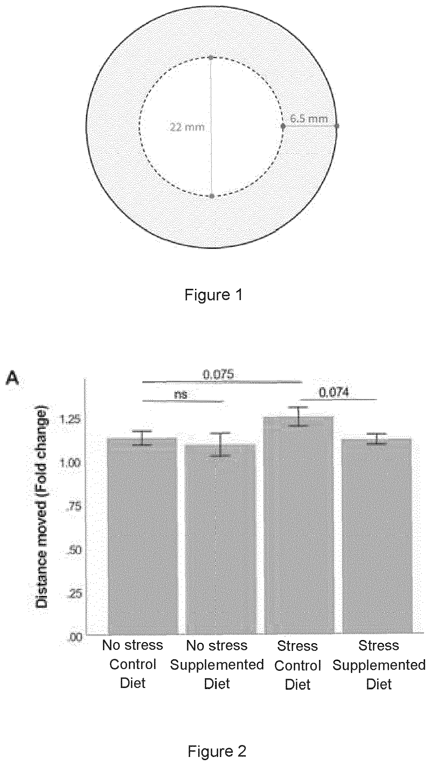

**Figure 1****.** Scheme of the well zones to evaluate thigmotaxis: peripheral area in gray and central area in white

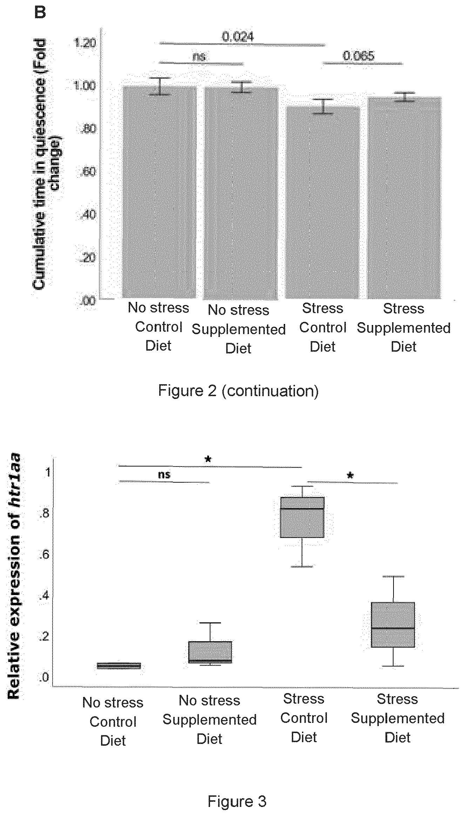

**Figure 2****.** Fold change between the dark phase and the light phase of each behavioral parameter related to anxiety. Data are represented as Mean ± S.E.M (Standard Error about the Mean); "ns" denotes non-significances (p > 0.05) by Mann Whitney test, while numbers on the bars indicate the p values between 0.05 and 0.1 as tendencies in the significance or p < 0.05 as statistically significant. (A) Total distance moved around the whole arena. (B) Cumulative time in quiescence.
**Figure 3****.** Relative expression of *htr1aa* (*5-hydroxytryptamine (serotonin) receptor 1A a*) using expression of *ef1a* as house-keeping gene. The boxes of the boxplot graph represent the data distribution between the quartile 1 and 3, the line shows the median of the data, and the whiskers indicate the minimum and maximum values. The asterisks (*) denote statistical significance by Student t-test, "ns" indicates non-significant data.

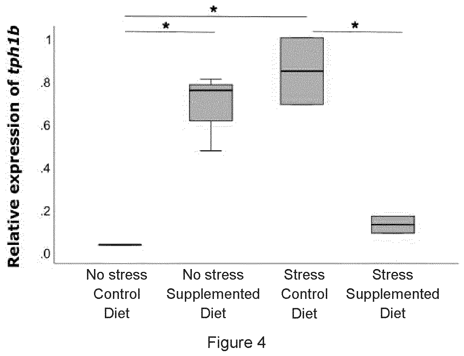

**Figure 4****.** Relative expression of *tph1b* (*tryptophan hydroxylase 1b*) using expression of *ef1a* as house-keeping gene. The boxes of the boxplot graph represent the data distribution between the quartile 1 and 3, the line shows the median of the data, and the whiskers indicate the minimum and maximum values. The asterisks (*) denote statistical significance by Student t-test.

### EXAMPLES

So as to contribute to a better understanding of the invention, and in accordance with a practical embodiment thereof, a preferred embodiment of the present invention is attached hereto as an integral part of this description.

### EXAMPLE 1: GUT-BRAIN AXIS EFFECT OF THE POSTBIOTIC COMPOSITION OF THE INVENTION IN ZEBRAFISH

### Objective

This study aimed to evaluate the effect of diet supplementation with the composition of the invention on a chronic stress zebrafish model. To do so, anxiety-related locomotor parameters were evaluated in 15 dpf (days post fertilization) zebrafish larvae after eight days of diet supplementation and daily application of unpredictable stress stimuli. Afterward, the diet effect on the expression of 3 genes involved in brain serotonin signaling metabolism, *tryptophan hydroxylase 1b (tph1b), solute carrier family 6 member 4a (slc6a4a)* and *5-hydroxytryptamine (serotonin) receptor 1A a (htr1aa),* were studied in these larvae. Zebrafish is an ideal model for conducting stress assays as anxiety is well-conserved in zebrafish, involving similar molecular and physiological mechanisms and behavioral responses to those studied in mammals, including humans.

### Material and methods

### Postbiotic composition of the invention:

A postbiotic composed of lysates from 17 strains/species of *Lactobacillus sp., Bifidobacterium sp., Bacillus sp, Saccharomyces sp.* and *Streptococcus sp.* was used. The composition of the extracts appears in Table 2 in an amount in percentage by weight over the total weight of the lysates of the composition and CFU percentage over the total CFUs.

**Table 2: Components of the postbiotic composition of the invention**

| Species/Strains | % (w/w) | % CFU |
|---|---|---|
| *Bacillus licheniformis* | 5 | 5,18 |
| *Bacillus mesentericus* | 2 | 1,55 |
| *Bacillus subtilis* | 5 | 5,18 |
| *Bifidobacterium bifidum* | 1 | 5,18 |
| *Bifidobacterium breve* | 10 | 0,52 |
| *Bifidobacterium lactis* | 3 | 7,77 |
| *Bifidobacterium longum* | 10 | 0,52 |
| *Lactobacillus acidophilus* | 2 | 2,07 |
| *Lactobacillus casei* | 2 | 10,36 |
| *Lactobacillus fermentum* | 2 | 2,07 |
| *Lactobacillus gasseri* | 5 | 0,26 |
| *Lactobacillus helveticus* | 5 | 0,26 |
| *Lactobacillus plantarum* | 1 | 5,18 |
| *Lactobacillus reuteri* | 3 | 3,11 |
| *Lactobacillus rhamnosus* | 3 | 15,54 |
| *Saccharomyces cereviseae* | 38 | 19,69 |
| *Streptococcus thermophilus* | 3 | 15,54 |

Unless otherwise specified, the composition of Table 2 was used in all experiments. The lyophilized powder of the composition of the invention was kept at 4°C until used.

### Animal care and embryo obtaining

Adult zebrafish were housed and maintained following standard procedures. Briefly, fish were maintained in 3-liter aquaria heated to 28.5 °C, with about 20 fish per tank and water continuously filtered. Water was maintained at pH 7-7.8, conductivity at 500-800 µS, and O₂ saturation between 80-100%. Water conditions were monitored and regulated conveniently. Fish were kept under a photoperiod of 14:10 hours light:dark. Parental fish were fed with ground dry pellets and live food following standard procedures.

### Materials Equipment and Reagents

### Materials

- Plastic tips
- 1.5 mL microtubes
- 6 well plates
- Pasteur pipets
- Petri dishes
- Protective equipment.

### Equipment

- Vortexes
- Autoclave
- Scales:
- Incubators at 28.5 °C
- -20 °C freezers
- 5 °C refrigerators
- Microcentrifuges
- Micropipette sets
- Dispensing pipette:
- Fume hoods
- Milli-Q water/ ELIX water system
- Microscope and microinjection systems
- Stereoscope
- Ice machine
- Leica stereo microscope
- DanioVision - EthoVision
- AriaMx Real-Time PCR System
- Ethovision XT v.9.0.726
- SPSS statistical software v26 (IBM SPSS Statistics)
- Agilent AriaMx Software v1.

### Reagents

Reagents used are shown in Table 3

**Table 3: List of reagents used**

| **Product** | **Storage conditions** | **Brand (Cat. N/Batch)** | **Vehicle** |
|---|---|---|---|
| Ampicillin sodium salt | 5 ± 3°C in solid state / -20°C in solution. | Sigma-Aldrich (A9518/BCCC0880) | MilliQ water |
| E3 Medium¹ | RT, tightly closed. | Biobide | N/A |
| Magnesium sulphate | RT, tightly closed. | Sigma-Aldrich (M5921/SLBX2919) | MilliQ water |
| Calcium chloride | RT, tightly closed. | Sigma-Aldrich (223506/BCCF9439) | MilliQ water |
| Potassium chloride | RT, tightly closed. | Sigma-Aldrich (31248/SZBF2720V) | MilliQ water |
| Sodium chloride | RT, tightly closed. | Sigma (S6191/SLCC5279) | MilliQ water |
| Methylene blue | RT, tightly closed. | ACROS Organics (229801000/A0331880) | MilliQ water |
| Hepes | RT, tightly closed. | Sigma (H3375/SLCH5583) | MilliQ water |
| DMSO | RT, protected from direct exposure to natural light. | Scharlab (SU01531000/21743805) | N/A |
| Tricaine | RT | Aldrich (E10521/WXBC5194V) | MilliQ water |
| Omniscript cDNA kit | -20 °C | Qiagen (40170796/172014209) | N/A |
| RNAqueous kit | 4 °C | Fisher (AM1912/ 01109586) | N/A |
| Brilliant III Ultra-Fast SYBR Green | -20 °C | Agilent (600882/006634683) | N/A |

| | | | |
|---|---|---|---|
| ¹ E3 content (0.16 mM MgSO₄, 0.4 mM CaCl₂, 0.17 mM KCI, 5 mM NaCl). E3 medium to be used in this Study is buffered to pH 7.2-7.6 with 10 mM HEPES. | | | |

### Chronic stress assay

Sample size: 24 embryos per condition (1 embryo per well). Although 40 larvae per condition were raised and subjected to the diet and stress protocol as explained below, in order to ensure the 24 individuals per experimental group at 15 dpf.

Control groups: diet control group with and without stressors, and diet supplemented with the composition of the invention without stressor.

Testing item: larvae were fed 3 times a day with the corresponding diet (control or the composition of the invention (postbiotic diet or supplemented diet)) from 6 dpf to 15 dpf, when zebrafish open their mouth.

Unpredictable chronic stress was implemented daily from 6 dpf to 14 dpf by turbulence exposure generated by an airstone.

Incubation conditions: natural cycles of 14 h Light - 10 h Darkness at 28.5 °C.

Locomotor parameters were evaluated at 15 dpf in an anxiety assay consisting in the light switch from 6 min of light to 4 min of darkness.

Experimental groups are shown in Table 4, experimental groups were distributed in 6-well plates for anxiety assay.

**Table 4: Experimental groups**

| **Experimental group** | **Diet** | **Stress exposure** |
|---|---|---|
| #1 | Control diet | No stress |
| #2 | Supplemented diet | No stress |
| #3 | Control diet | Chronic stress |
| #4 | Supplemented diet | Chronic stress |

### PROCEDURE

### Embryo production

Embryos for the development of the example were obtained and collected following regular protocols in the field and raised in E3 medium with ampicillin (100 µg/mL) and methylene blue (0.0001%). Synchronized embryos were supplied to the laboratory and sorted into 4 different groups for further experimental conditions of diet and stress exposure. Embryos were kept in the incubator at 28.5 °C until 5 dpf.

Due to the expected mortality during the critical period from 6 to 15 dpf, an exceed of larvae were raised to ensure an experimental group size of 24 larvae at 15 dpf. Thus, 40 larvae per experimental group at 5 dpf were introduced in 3 L tanks in a static system at the maximum ratio of 20 larvae/l (i.e. one tank per experimental group). Group size were similar among experimental groups to avoid density effects on the stress. They were maintained under natural lighting conditions of 14 h Light - 10h Darkness and 10 % of the water was manually replaced every day to ensure water quality. Diet was scheduled and administrated from 6 to 15 dpf.

### Preparation and administration of the diet containing the test items

The supplemented diet comprised 50 % standard diet plus 50 % the postbiotic composition of the invention and was prepared by thorough mixing of the powders, and then, spraying-coating with vegetable oil. Control diet was only prepared by spraying vegetable oil on standard larvae food (Sera micron). Diets were left to air-dry and kept in airtight plastic containers at 4°C until use.

Larvae from 6 to 14 dpf were fed with 18 mg/day/tank of the corresponding dry food distributed in 3 meals, either diet supplemented with the active ingredients to test or the control diet, according to the experimental group. The 3 feeding meals were scheduled at the same times of the day (9:00, 12:00, and 15:00) to not induce extra stress in the larvae.

### Chronic stress induction

From 6 dpf to 14 dpf, only half of the larvae from each diet group were daily subjected to chronic unpredictable stress. Hence, a stress stimulus was administrated three times a day at random times between 8 h and 17 h to keep unpredictability. Stress stimulus consisted in larvae disturbance by turbulence exposure generated by airstones. Thus, larvae in the stressed groups were exposed to chronic stress for 8 days, while larvae in the control groups were raised in the same conditions without stressor exposure.

### Anxiety assay

Larvae received their last meal before 10 am on the day when the anxiety assay was performed (15 dpf). After 12 am on the 15th dpf, 24 larvae per experimental group were individually transferred to 6-well plates in 3 mL water system. Plates were introduced in the DanioVision observational chamber (Noldus) at 28°C. There, larvae were acclimated for 15 min under the lights on. Afterward, larvae were tracked for 10 min: 6 min under light plus for 4 min in darkness after an abrupt light switch-off, in order to evaluate the response to the light switch. Parameters related to anxiety such as total distance moved, thigmotaxis (i.e. ratio between the distance moved in the peripheral well area and the distance moved in the total well area), and freezing behavior (i.e. duration and frequency of quiescent bouts), were obtained by Ethovision software (Noldus). The peripheral area was determined by the larvae length accordingly to Schnörr et al. (2012). At 15 dpf, zebrafish larvae length is estimated in 6.2 mm approximately, thus, 6.5 mm was the distance between the well wall and the central area perimeter (**Figure 1**).

After the trial was completed, larvae were observed under the stereoscope to identify possible gross developmental defects that could affect to their normal locomotion, in which case, they would be excluded from the behavioral further analyses.

### Quantification of genes expression

Once the anxiety assay was accomplished, the expression of 3 genes involved in brain serotonin signaling metabolism, *tryptophan hydroxylase 1b (tph1b) solute carrier family 6 member 4a (slc6a4a)* and *5-hydroxytryptamine (serotonin) receptor 1A a (htr1aa),* was assessed. To do so, 15 dpf larvae were washed, collected, and euthanized by immersion in overdoses of tricaine (0.25 mg/mL). Then, the heads of the euthanized larvae were excised, and 3 pools of 8-12 heads (depending on the survival) from larvae belonging to the same experimental group were collected from each condition and stored at -20°C. RNA was extracted with RNAqueous kit (Ambion) and retrotranscribed into cDNA using the OmniScript kit (Qiagen), following the manufacturer's instructions for both kits. Then, cDNA samples were assessed in triplicates by semi-quantitative real-time PCR using SYBR green and gene-specific primers in an AriaMx Real-Time PCR System (Agilent Technologies). The gene-specific primers for *tph1b* and *htr1aa,* and eukaryotic *translation elongation factor 1 alpha 1, like 1 (ef1a)* as a house-keeping gene were designed and their sequence and product size are indicated in Table 5:

**Table 5: Primers used**

| **Target gene** | **Primer sequence (5' → 3')** | | **Product size (bp)** |
|---|---|---|---|
| *ef1a* | Forward | ACCTACCCTCCTCTTGGTCG | 183 |
| | Reverse | AGAACACGCCGCAACCTTTG | |
| *tph1b* | Forward | CGCAGATCATCCGGGATTCA | 73 |
| | Reverse | ACTCATGGCAAGATCCGCAA | |
| *slc6a4a* | Forward | TGTGTAAGTGCATCCGGCAG | 196 |
| | Reverse | ATCAGCAACACCAGGTACGG | |
| *htr1aa* | Forward | CTACTCAACTTTCGGGGCGT | 145 |
| | Reverse | CACCGCCAAGCATTTATCCG | |

### Evaluation, and interpretation of the results

### Data analysis

Raw data regarding larvae locomotor activity in 1-minute time-bins was firstly analyzed by Ethovision XT software to obtain the following locomotor parameter from both, light and dark phases, separately:
- total distance moved
- freezing behavior as duration of quiescent bouts

The data of each variable described above were statistically compared between the different experimental groups by a statistical test, in order to determine the effect of the diet on the anxiety-related locomotor parameters.

Raw data from the expression level of genes involved in brain serotonin signaling metabolism (tph1b and htr1aa) were normalized by the ef1a expression (used as house-keeping gene) from each sample. Relative gene expression was statistically compared between the experimental groups.

The graphs showed the mean and standard mean error, and the statistically significant differences were annotated as "*" (p < 0.05), tendencies (0.05 < p < 0.1), and "ns" (non-significant data).

### Results

Larvae behavior during the light phase was considered as the baseline, while the response to the stress was expected during the dark phase. The differences for each locomotor parameter between different experimental groups were considered significant if the p < 0.05. Also, the p values between 0.1 and 0.05 could have been taken into account as an indicator of tendency.

The diet was considered to induce an alteration in the gut-brain modulation of chronic stress if the observed locomotor parameters presented differences between the larvae fed with the supplemented diet and larvae fed with the control diet, both subjected to chronic stress. The criteria to classify the diet as anxiolytic was evaluated based on the combination of the following outputs: a decrease in the thigmotaxis, reduction of freezing behavior, and hypoactivity. The opposite effects were the criteria to classify the diet as anxiogenic. The presence of no significant behavioral alterations was the criterium to classify the diet as having "no effect" on the modulation of chronic stress.

Differences between different experimental groups in the gene expression for each of the 3 analyzed genes were considered significant if the p < 0.05. Also, the p values between 0.1 and 0.05 could have been taken into account as an indicator of tendency. The alteration of the gene expression level in larvae fed with the supplemented diet with respect to larvae fed with the control diet (both subjected to chronic stress) was the criterium to determine if the diet affects the serotonin signaling metabolism.

### Anxiety assay

Larvae from 6 to 14 dpf were subjected to 4 different experimental conditions: no stress and control diet, no stress and supplemented diet, chronic stress and control diet, and chronic stress and supplemented diet. At 10 am, 15 dpf larvae were collected from the tanks and dispended into the 6-well plates where the anxiety assay was performed. After 12 am, larvae were subjected to 15 min of habituation, and then, tracked during 6 min of light plus 4 min of darkness. After the trial was completed, larvae were observed under the stereoscope to identify possible gross developmental defects that could affect their normal locomotion. Thus, one larva from the chronic stress and control diet was found dead and excluded from further analyses.

Behavioral data were separately calculated during the last 4 min of light and during the 4 min of darkness. Thus, the last 4 min under light served as baseline activity to normalize the values corresponding to the 4 min under darkness in order to study the fold change caused by the light switch. The following parameters related to anxiety were obtained: total distance moved in the whole arena, cumulative time in quiescence. Extreme outliers identified by Tukey's Hinges were removed from the data analysis. Since data for any of the studied variables in any experimental groups presented normal distribution (Shapiro-Wilk test, p < 0.05), paired comparisons for 2 independent samples were performed by Mann Whitney test to compare the behavioral data.

A tendency to a greater increase (p = 0.075) in the total distance moved in the whole arena after the light switch was observed in the larvae fed with control diet and exposed to chronic stress than in those without stressor (**Figure 2A****)**. However, when larvae subjected to chronic stress were fed with the supplemented diet, this increase in the total distance moved tended to be reduced (p = 0.074).

The change in the cumulative time of quiescent bouts (**Figure 2B**) caused by the light switch was significantly reduced (p = 0.024) in stressed larvae than in non-stressed larvae, both fed with control diet.

In table 6, the measurements of the results shown in Figure 2A and 2B are shown:

**Table 6: measurements**

| **Condition** | Total distance (Fig 2A) | Cumulative time of quiescent botus (Fig 2B) |
|---|---|---|
| | Average | Average |
| No stress + Control Diet | 1,13 | 0,99 |
| No stress + Supplemented Diet | 1,09 | 0,99 |
| Chronic Stress + Control Diet | 1,25 | 0,9 |
| Chronic Stress + Supplemented Diet | 1,12 | 0,94 |

The addition of supplemented diet to the larvae under stress stimulus allows to restore the total distance moved compared to the levels of the larvae fed with control diet and no stress stimulus. While, the addition of supplemented diet to the larvae under stress stimulus allows to partially restore the cumulative time of quiescent botus

Finally, no effect of the supplemented diet with the postbiotic composition of the invention was observed on the non-stressed larvae in any of the studied behavioral parameters when compared with those fed with control diet (Figure 2), which indicates that the postbiotic composition of the invention does not negatively affect Zebrafish.

### Quantification of gene expression

The brain expression of 2 genes involved in serotonin signaling metabolism, *tph1,* and *htr1aa,* was assessed. Three pools of 8 heads of 15 dpf larvae were collected from each of the 4 experimental conditions (no stress and control diet, no stress and supplemented diet, chronic stress and control diet, and chronic stress and supplemented diet). The expression of the genes of interest was analyzed by quantitative real-time PCR. Thus, the relative gene expression level of each sample was calculated by the 2^{-ΔΔCT} method, using ef1a as house-keeping gene to normalize the expression of the gene of interest (tph1b, slc6a4a, and htr1aa). The second normalization in the 2^{-ΔΔCT} calculations was performed using the sample with the lowest value within each gene to later analyze the statistical differences between the different experimental groups. Extreme outliers were removed from the data analysis. After confirming the normal distribution of the data (Shapiro-Wilk test, p > 0.05) in all cases, the data were analyzed by Student t-test for independent samples preceded by Levene's test to check the homogeneity of variance.

When comparing the control diet groups, the relative expression of *htr1aa* increased (p = 0.018) in the group subjected to chronic stress (**Figure 3**). However, when comparing both groups under chronic stress, larvae fed with supplemented diet decreased the htr1aa expression (p = 0.044) in comparison with those larvae fed with control diet. Moreover, the supplemented diet did not alter the htr1aa expression in the non-stressed larvae (p = 0.410).

In contrast, the chronic stress stimulus did not modify the relative expression of *slc6a4a* when comparing both groups fed with the control diet. Likewise, when comparing both groups under chronic stress, the administration of probiotic produce a slight reduction in the levels of *slc6a4a* expression, although this tendency was not statistically significant.

The *tph1b* expression increased (p = 0.035) under the chronic stressor when comparing larvae fed with the control diet (**Figure 4**). Also, the administration of supplemented diet decreased (p = 0.047) the *tph1b* expression when comparing both groups subjected to chronic stress. However, the supplemented diet under no chronic stress increased (p = 0.025) the expression of this gene in comparison with the control diet feeding. It is important to remark that the measurements of the condition "no stress + supplemented diet" show the highest variability among all the measurements.

### Conclusions

The objective of this study was to evaluate the effect of the postbiotic composition of the invention in diet supplementation on zebrafish larvae exposed to chronic stress caused by unpredictable daily stress stimuli. Thus, zebrafish larvae were subjected to 4 different experimental conditions: no stress and control diet, no stress and supplemented diet, chronic stress and control diet, and chronic stress and supplemented diet. To do so, the corresponding diet and the stress stimuli were applied from 6 dpf. At 15 dpf, larvae were collected and placed in 6-well plates for the anxiety assay which consisted of 15 min of habituation in light and tracking of 6 min under light plus 4 min under darkness. Then, the change of anxiety-related locomotor parameters provoked by the light switch was evaluated. Chronic stress protocol applied provoked a tendency to increment the stress reaction after the light switch. Hence, a tendency to greater increase the total distance moved in the whole arena and a significantly stronger reduction of the cumulative time in quiescence was observed in larvae fed with control diet and subjected to stress than in those without stress stimuli. However, these reactions tended to be milder when larvae were fed with the supplemented diet, pointing to an anxiolytic effect of the experimental diet. Finally, the supplemented diet did not influence the larvae reaction with no chronic stress.

Subsequently, the diet effect on the brain expression of 3 genes involved in serotonin signaling metabolism, *tryptophan hydroxylase 1b (tph1b), solute carrier family 6 member 4a (slc6a4a),* and *5-hydroxytryptamine (serotonin) receptor 1A a (htr1aa),* were studied in these larvae after the anxiety assay was performed. Gene expression data differ from each gene of interest. Thus, the chronic stress applied increased the brain expression of *htr1aa* and *tph1b* in larvae fed with the control diet. Moreover, the supplemented diet decreased this expression when compared to chronically stressed larvae. The *slc6a4a* brain expression was similar in all experimental groups, although a slight decreased expression of said gene was observed in larvae fed with the diet comprising the postbiotic composition of the invention.

Statistical tendencies of behavioral data (total distance moved and cumulative time of quiescence) pointed to classify the diet as anxiolytic. Besides, regarding the serotonin signaling metabolism, the supplemented diet decreased the brain expression of *htr1aa, tph1b* and slightly in the case of *slc6a4a* in chronically stressed larvae.

Altogether, the data demonstrates the therapeutic use of the postbiotic composition of the invention as an anxiolytic under stress stimuli.

### LIST OF SEQUENCES

(*ef1a Forward*) SEQ_ID No 1: ACCTACCCTCCTCTTGGTCG
(*ef1a Reverse*) SEQ_ID No 2: AGAACACGCCGCAACCTTTG
(*tph1b Forward*) SEQ_ID No 3: CGCAGATCATCCGGGATTCA
(*tph1b Reverse*) SEQ_ID No 4: ACTCATGGCAAGATCCGCAA
(*slc6a4a Forward*) SEQ_ID No 5: TGTGTAAGTGCATCCGGCAG
(*slc6a4a Reverse*) SEQ_ID No 6: ATCAGCAACACCAGGTACGG
(*htr1aa Forward*) SEQ_ID No 7: CTACTCAACTTTCGGGGCGT
(*htr1aa Reverse*) SEQ_ID No 8: CACCGCCAAGCATTTATCCG

## Claims

1. A postbiotic composition that comprises lysates of probiotic microorganisms of the genus *Bacillus, Bifidobacterium, Lactobacillus, Saccharomyces* and *Streptococcus* for use in the treatment and/or prevention of psychiatric disorders in a subject.

2. The postbiotic composition for use, according to the preceding claim, wherein the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus Bacillus paralicheniformes,* and combinations thereof.

3. The postbiotic composition for use, according to any of the preceding claims, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof.

4. The postbiotic composition for use, according to any of the preceding claims, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof.

5. The postbiotic composition for use, according to any of the preceding claims, wherein the lysates of yeast genus *Saccharomyces* are of the species that are selected from the group consisting of *Saccharomyces cerevisiae, Saccharomyces boulardii,* and combinations thereof.

6. The postbiotic composition for use, according to any of the preceding claims, wherein the bacterial lysates of the genus *Streptococcus* are of the species that are selected from the group consisting of *Streptococcus thermophilus, Streptococcus salivarius,* and combinations thereof.

7. The postbiotic composition for use, according to the preceding claims 1 to 6 wherein the composition comprises lysates of probiotic microorganisms of *Bacillus licheniformis, Bacillus subtilis, Bacillus mesentericus, Bifidobacterium bifidum, Bifidobacterium breve, Bifidobacterium lactis, Bifidobacterium longum, Lactobacillus acidophilus, Lactobacillus casei, Lactobacillus fermentum, Lactobacillus gasseri, Lactobacillus helveticus, Lactobacillus plantarum, Lactobacillus reuteri, Lactobacillus rhamnosus, Saccharomyces cerevisiae* and *Streptococcus thermophilus*

8. The postbiotic composition for use, according to the preceding claim, wherein the percentage by weight of *Bacillus licheniformis* and *Bacillus subtilis* is approximately the same, and is higher than that of *Bacillus mesentericus.*

9. The postbiotic composition for use, according to any of the preceding claims 7 or 8, wherein the percentage by weight of *Bifidobacterium longum* and *Bifidobacterium breve* is approximately the same, and is higher than that of *Bifidobacterium lactis* which is higher than that of *Bifidobacterium bifidum.*

10. The postbiotic composition for use, according to any of the preceding claims 7 to 9, wherein the percentage by weight of *Lactobacillus gasseri,* and *Lactobacillus helveticus* is approximately the same, and is higher than that of *Lactobacillus reuteri* and *Lactobacillus rhamnosus,* which is also approximately the same and is higher than that of *Lactobacillus acidophilus, Lactobacillus casei* and *Lactobacillus fermentum,* which is also approximately the same and is higher than that of *Lactobacillus plantarum.*

11. The postbiotic composition for use, according to claim 1, that comprises lysates of probiotic microorganisms in a percentage amount by weight with respect to the total weight of the lysates of microorganisms of the composition:
- 6% to 20% of bacterial lysates of the genus *Bacillus;*
- 8% to 40% of bacterial lysates of the genus *Bifidobacterium;*
- 8% to 40% of bacterial lysates of the genus *Lactobacillus;*
- 20% to 60% of yeast lysates of the genus *Saccharomyces;*
- 0.5% to 8% of bacterial lysates of the genus *Streptococcus.*

12. The postbiotic composition for use, according to the preceding claim, that comprises lysates of probiotic microorganisms in a percentage amount by weight with respect to the total weight of the lysates of microorganisms of the composition:
- 6% to 20% of bacterial lysates of *Bacillus licheniformis, Bacillus mesentericus* and *Bacillus subtilis;*
- 8% to 40% of bacterial lysates of *Bifidobacterium bifidum, Bifidobacterium breve, Bifidobacterium lactis* and *Bifidobacterium longum,*
- 8% to 40% of bacterial lysates of *Lactobacillus acidophilus, Lactobacillus casei, Lactobacillus fermentum, Lactobacillus helveticus, Lactobacillus gasseri Lactobacillus plantarum, Lactobacillus reuteri* and *Lactobacillus rhamnosus;*
- 20% to 60% of yeast lysates of *Saccharomyces cerevisiae;*
- 0.5% to 8% of bacterial lysates of *Streptococcus thermophilus.*

13. A pharmaceutical formulation comprising an effective pharmaceutical amount of the composition according to any of the claims 1 to 12, and at least one pharmaceutically acceptable excipient for use in the treatment of psychiatric disorders in a subject.

14. A food supplement comprising the composition of the invention for use in the treatment of psychiatric disorders in a subject.

15. The composition for use, according to any of the claims 1 to 12, the pharmaceutical formulation for use, according to claim 13 and/or the food supplement for use, according to claim 14, wherein the psychiatric disorder is selected from the group consisting of: an obsessive compulsive disorder, a depressive disorder, a schizophrenia disorder, a schizotypal disorder, an anxiety disorder, substance abuse, and/or an avolition disorder.
